# EUROPÄISCHE PATENTSCHRIFT

(11) **EP 1 595 960 B1**
(45) Veröffentlichungstag und Bekanntmachung des Hinweises auf die Patenterteilung: **10.09.2008**
(21) Anmeldenummer: 05012873.5
(22) Anmeldetag: 29.05.1999
(51) Int. Cl.: C12Q 1/68

(54) **DNA-Nachweis über einen Strangreassoziationskomplex**
DNA detection via complex of reassociated strings
Détection de l'ADN par la formation d'une complexe des brins réassociés

(30) Priorität: 04.06.1998 DE 19824900
(43) Veröffentlichungstag der Anmeldung: 16.11.2005
(62) Teilanmeldung aus: 99110458.9
(73) Patentinhaber: Roche Diagnostics GmbH, 68305 Mannheim (DE)
(72) Erfinder: Weindel, Kurt, Dr., 82407 Wielenbach-Hardt (DE); Brand, Joachim, 82380 Peissenberg (DE)
(74) Vertreter: Herren, Barbara

(56) Entgegenhaltungen:
- EP-A- 0 357 011
- WO-A-95/25538
- WO-A-97/47640
- DE-A1- 4 041 608

## Beschreibung

Gegenstand der Erfindung ist ein Verfahren zum Nachweis von Nucleinsäuren unter Verwendung der Amplifikation der Nucleinsäure mit Hilfe markierter Primer sowie Nachweis des Amplifikates mit Hilfe einer Fangsonde.

Biospezifische Binding Assays, die mittels molekularer Erkennungsmechanismen den Nachweis bestimmter Analyte bzw. Analytmerkmale ermöglichen, sind aus Diagnostik und Bioanalytik nicht mehr wegzudenken. Dabei haben sich in den letzten Jahren neben den Immunoassays und Rezeptor Ligand-Assays Hybridisierungsassays fest etabliert. In Hybridisierungsassays wird das Prinzip der Paarung von Nukleobasen (A::T; G:::C) zur molekularen Erkennung bestimmter Analytnukleinsäuren (z. B. DNA, RNA) durch Sonden mit gewünschter Spezifität ausgenutzt. So sind z. B. mit Oligonucleotidsonden, die aus 18 - 20 Nucleotiden in ausgewählter Sequenz aufgebaut sind, eindeutige Erkennungen selbst über das menschliche Genom hinweg möglich.

Eine interessante und vielversprechende Erweiterung erfahren Hybridisierungsassays (= probe based assays) durch sogenannte NA Chip-Technologien. Hier sind auf einem Testträger mindestens 2, meist mehrere bis sehr viele Sonden (probes) mit unterschiedlichen Sequenzen und somit unterschiedlicher Spezifität in einem geometrischen Muster in getrennten Bereichen gebunden, so daß entsprechend viele Hybridisierungsreaktionen zwischen Sonden und Nucleinsäureanalyt-Segmenten bzw. verschiedenen Nucleinsäure-Analyten parallel durchgeführt werden können. Unter geeigneten Reaktionsbedingungen, z. B. Sequenzauswahl, Puffermilieu, Salzgehalt und vor allem Inkubations- und Waschtemperatur, gelingt es, nur solche Hybridisierungskomplexe festphasengebunden zu halten, bei denen alle in der Oligonucleotidsonde enthaltenen Nucleotide komplementär sind zu den entsprechenden Nucleotiden im Analytmolekül, so daß die volle Bindungsstärke resultiert. Man spricht dann von vollständiger Basenpaarung (perfect match, PM). Hybridisierungskomplexe, die Fehlpaarungen (mismatches, MM) enthalten, werden unter solchen Bedingungen abgelöst. Unter optimalen Bedingungen können sogar Komplexe mit vollständigen Basenpaarungen von Komplexen mit 1-Punkt-Fehlpaarungen (single base transitions) eindeutig unterschieden werden. Da dies parallel unter Anwendung eines geometrischen Fangsondenmusters (array) auf der Festphase geschieht, spricht man von probe array testing.

Die Fangsonden können alle eine konstante Länge (Anzahl Nucleotidbausteine) haben, oder die Oligo-Länge kann dem GC-Gehalt umgekehrt proportional angepaßt sein. Im ersten Fall kann eine gemeinsame Schmelztemperatur Tm für alle vollständig gepaarten Hybridisierungskomplexe erreicht werden durch Pufferadditive, die z. B. AT-Bindungen so verstärken, daß Tm unabhängig von der Nucleobasensequenz und allein abhängig von der Oligo-Länge wird. Beispiele für solche Additive sind Tetramethylammonium-chlorid (TMAC) und Tetraethylammonium-bromid. Im zweiten Fall bewirkt die angegebene Längenadaptation eine Tm-Nivellierung. Die Fangsonden können chemisch unterschiedliche Backbones haben, welche die spezifitätsvermittelnden Nucleobasen tragen; z. B. Deoxyribosyl-phosphodiester-Stränge (=> DNA), Ribosyl-phophodiester-Stränge (=> RNA), oder aber einer nicht natürlichen Substanzklasse angehören, z. B. N-(2-Aminoethyl)glycyl- oder N-(2-Aminoethyl)glutamyl-Stränge (=> PNA, WO 92/20702).

Probe array testing ist für viele molekularbiologische oder diagnostische Anwendungen interessant. Dazu gehört Multi-Pathogen Testing (simultaner Nachweis verschiedener Krankheitserreger auf Genebene), (Sub-)Typisierung von Organismen, Analyse von genetischer Diversität (Polymorphismen, Mutationen), Sequenzierung von Genen oder Genomen, etc..

Nucleinsäuren sind vergleichsweise komplexe Analyte, die in der Regel zuerst isoliert, dann amplifiziert, und im Falle von DNA einzelsträngig gemacht (denaturiert) werden müssen, bevor sie in einem probe based assay oder probe array testing eingesetzt werden können. Von diesem Aufarbeitungsgang her und der Tatsache, daß komplementäre Nucleobasen auch eine Neigung zur Basenpaarung innerhalb ein und desselben Stranges haben, ergeben sich einige typische Schwierigkeiten, wie z. B. variable Analyt-Titer in der Reaktionslösung aufgrund schwankender Effizienz bei der Isolierung oder Amplifikation, suboptimale Denaturierungseffizienz, Reassoziation der Einzelstränge einer DNA zum ursprünglichen Doppelstrang als Konkurrenz zur Hybridisierung eines Einzelstrangs mit der Sonde, strang-interne Hybridisierung (Sekundärstrukturbildung, z. B. Haarnadelschleifen oder Kreuzformationen) als Konkurrenz zur Sondenhybridisierung. Diese ist umso stärker, je höher der palindrome Index, d. h. die Selbstkomplementarität, eines DNA- oder RNA-Stranges ist.

Besonders die letzten beiden Phänomene bestimmen wesentlich die Zugänglichkeit der dem Test zugrundeliegenden Sequenzregion des Analyten und limitieren dadurch die Gesamt-Performance über ein Array von Fangsonden hinweg.

Bei der Amplifikation der Analytnukleinsäure bedient man sich meistens der sog. PCR-Methode (polymerase chain reaction, US-A-4,683,202). Dabei hat man die Möglichkeit, gleich bei der Amplifikation eine nachweisbare Gruppe einzubauen, z. B. ein Digoxigenin-Derivat (DIG-Markierung, EP-B-0 324 474). Dies kann geschehen, indem man im Nucleosidtriphosphat-Mix ein Teil des dTTP ersetzt durch DIG-dUTP.

In DE-A-3807994 (US-A-5,476,769) ist ein Verfahren beschrieben, in dem detektierbar markierte Amplikonstränge an einer immobilisierbaren Fangsonde hybridisiert und die gebildeten Hybride nachgewiesen werden.

In EP-A-0 079 139 ist das sogenannte Sandwich-Hybridisierungsverfahren beschrieben, in dem eine nachzuweisende Nucleinsäure durch Hybridisierung mit einer Fangsonde und einer Nachweissonde, die zu unterschiedlichen Bereichen der Nucleinsäure komplementär sind, nachgewiesen werden.

WO 95/25538 offenbart Verfahren und Kits zur Generierung und Detektion polymorpher Restriktionsschnittstellen in Nukleinsäuren, wobei Nukleinsäuren nach Amplifikation mit einer Restriktionsendonuklease gespalten und an eine Festphase gebunden werden.

Aufgabe der vorliegenden Erfindung war es, Hybridisierungsassays, die auf einer Abfangreaktion von Amplifikaten, die unter Einbau einer Markierung erzeugt wurden, zu verbessern, insbesondere hinsichtlich ihrer Diskriminierung zwischen zwei oder mehr Nukleinsäuren mit sehr nahe verwandten Sequenzen.

Ein Gegenstand der vorliegenden Erfindung ist ein Verfahren zum selektiven Nachweis einer Nucleinsäure enthaltend die Schritte Amplifikation der Nucleinsäure oder eines Teils davon mit Hilfe zweier Primer, von denen einer mit einem Strang der nachzuweisenden Nucleinsäure und der andere mit einem hierzu komplementären Strang hybridisieren kann und von denen mindestens einer eine nachweisbare Markierung gebunden enthält, unter Bildung jeweils eines Verlängerungsproduktes dieser Primer in einer Reaktionsmischung, Bindung einer Fangsonde an eines dieser Verlängerungsprodukte unter Ausbildung eines Hybridisierungskomplexes, der die Fangsonde und mindestens dieses Verlängerungsprodukt enthält, Abtrennung des an die Fangsonde gebundenen Verlängerungsproduktes von nicht gebundenen Anteilen der Reaktionsmischung, und Bestimmung der an die Fangsonde gebundenen nachweisbaren Markierung, wobei die Fangsonde so gewählt wird, daß sie mit dem Strang des Verlängerungsproduktes binden kann, der auch mit dem durch Verlängerung des markierten Primers gebildeten Verlängerungsprodukt hybridisieren kann.

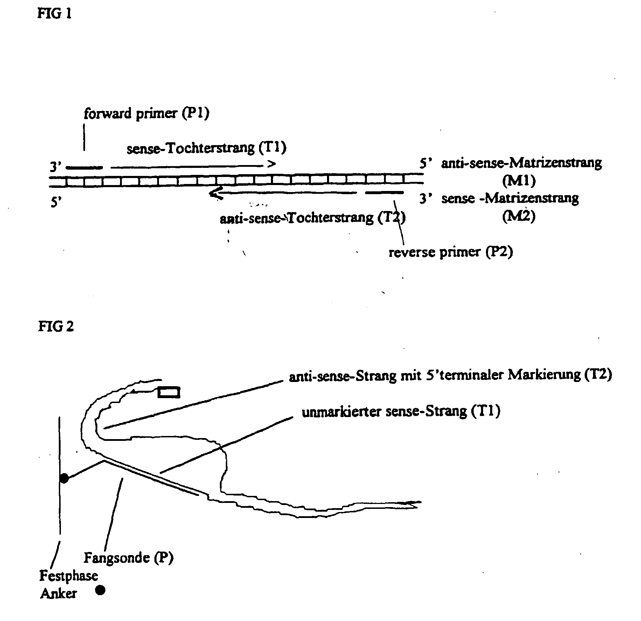

In FIG 1 ist schematisch die Benennung der einzelnen Komponenten der Amplifikation im Sinne der Erfindung gezeigt. Hierbei bedeuten:
- P 1: forward-Primer
- P2: reverse-Primer
- T1: sense-Tochterstrang (sense-Verlängerungsprodukt)
- T2: anti-sense Tochterstrang (anti-sense-Verlängerungsprodukt)
- M 1: anti-sense Matrizenstrang (Templat); Strang der nachzuweisenden Nucleinsäure
- M2: sense-Matrizenstrang (Templat); Gegenstrang zur nachzuweisenden Nucleinsäure

In FIG 2 ist schematisch ein schon festphasengebundener Komplex mit den Strängen T1 (unmarkiert), T2 (5'-terminal markiert) und der festphasengebundenen Sonde P gezeigt. Das Rechteck ist eine detektierbare Markierung.

In FIG 3 ist die Lage der Regionen mit Mutationen im Segment gezeigt.

In FIG 4 ist ein Faltungsvorschlag für das rpo-β-Gensegment gezeigt.

In FIG 5 ist ein Vergleich zwischen der idealen Situation (stäbchenförmige DNA) mit der realen Situation A (gefaltet) gezeigt. Es wird deutlich, daß die Zugänglichkeit der terminalen Markierung real stark beeinträchtigt ist. Diese Beeinträchtigung wird nach dem Vorgehen gemäß der vorliegenden Erfindung reduziert bzw. vermieden.

Ein Kern der vorliegenden Erfindung ist die überraschende Beobachtung, daß die beste Diskriminierungsschärfe (Unterscheidung PM gegenüber MM) dann gelingt, wenn die festphasengebundenen Fangsonden komplementär antiparallel zum sense-Strang konzipiert sind, welcher in der PCR ausgehend von einem unmarkierten forward-Primer synthetisiert wird, die Nachweisreaktion aber auf den anti-sense-Strang zugreift, welcher in der PCR ausgehend von einem mit einer nachweisbaren Gruppe markierten reverse-Primer synthetisiert wird, so daß der die Spezifität (Diskriminierungsschärfe) bestimmende Sondenhybridisierungskomplex indirekt über den DNA-Strangreassoziationskomplex nachgewiesen und ggfs. quantifiziert. Umgekehrt kann die Fangsonde auch komplementär zum anti-sense-Strang sein und der Nachweis erfolgt über Reassoziation mit dem via markierten forward-Primer nachweisbaren sense-Strang.

Dies wirkt sich zwar auch bei Einzelhybridisierungen aus, besonders positiv ist es jedoch im Zusammenhang mit parallel durchgeführten Hybridisierungsassays, d. h. (D)NA probe array testing.

Unter einem selektiven Nachweis wird ein Nachweis verstanden, der eine Nucleinsäure mit einer Zielsequenz, die sich nur geringfügig von einer Nicht-Zielsequenz unterscheidet, aus einer Probe auswählt und bindet, die eine Vielzahl von Nucleinsäuren enthält, welche die Zielsequenz nicht aufweisen, und unter denen sich auch die Zielsequenz befinden kann oder befindet. Die Bindung findet durch Paarung komplementärer Basen, wie A und T bzw. U sowie G und C, einer Sonde und der nachzuweisenden Nucleinsäure statt. Hierzu ist die Sequenz der Sonde hochgradig komplementär, bevorzug 100 % komplementär, zu der Zielsequenz auf der Nucleinsäure, insbesondere bezüglich solcher Basen, in denen sich die Zielsequenz von Nicht-Zielsequenzen unterscheidet. Die Sondensequenz ist bevorzugt zwischen 8 und 36 nt, besonders bevorzugt zwischen 16 und 20 nt lang. Die chemische Struktur der Sonde, insbesondere in dem nicht-Nucleobasenteil, z. B. dem sogenannten Backbone, kann relativ frei gewählt werden, vorausgesetzt, die selektive Bindung mit der Zielsequenz ist noch möglich. Neben den (bevorzugten) Oligonucleotiden (als Backbone dient das natürliche oder durch Anknüpfung von Gruppen modifizierte Zucker-Phosphat-Grundgerüst) haben sich in jüngster Zeit auch die sogenannten Peptid-Nucleinsäuren (PNA gemäß WO 92/20702, Backbone aufgebaut unter Einbau von künstlichen Aminosäuren) bewährt. Als Sonden sind auch Moleküle mit gemischten Grundgerüst-Einheiten geeignet (WO 95/14706 oder EP-A-0 672 700).

Eine Fangsonde, deren Funktion in der sequenzspezifischen Immobilisierung der Reaktionskomplexe an die Festphase besteht, kann beispielsweise dann mit dem Strang des Verlängerungsproduktes binden, der auch mit dem durch Verlängerung des markierten Primers gebildeten Verlängerungsproduktes hybridisieren kann, wenn sie zu einer Sequenz auf diesem Verlängerungsprodukt komplementär ist. Diese Sequenz ist dann die Zielsequenz. Sie ist bei der nachzuweisenden Nucleinsäure entweder bekannt, kann durch Sequenzierung ermittelt werden oder wird erst durch die Bindung an eine oder mehrere Fangsonde(n) gemäß der vorliegenden Erfindung definiert und bestimmt.

Die Zielsequenz wird bevorzugt so gewählt, daß sie zwischen den aufeinanderzuweisenden "inneren" Enden der Primer liegt. Die Sequenz der Zielsequenz wird so gewählt, daß sie sich von Sequenzen nicht-nachzuweisender Nucleinsäuren unterscheidet, insbesondere in ihrer Basenabfolge. Die Unterschiede können bedingt sein beispielsweise durch Mutationen (Einzel-Basen-Austausche, aber auch mehrere), Deletionen, Insertionen oder Polymorphismen.

Ein Fachmann kann die geeigneten Zielsequenzen beispielsweise durch Sequenzrecherche und Sequenzvergleich in den bekannten Sequenzdatenbanken auffinden. Im Sinne einer bevorzugten Ausführungsform der Erfindung, in der mehrere Sequenzen, Sequenzunterschiede, Mutationen, Polymorphismen oder ähnliches nachgewiesen werden, liegen die Zielsequenzen bevorzugt innerhalb eines Bereiches, der mit Hilfe eines einzigen Primerpaares (ein reverse- und ein forward-Primer) amplifiziert werden kann. Es ist jedoch prinzipiell auch möglich, mehr Primer zu verwenden, insbesondere wenn sie alle zu Amplifikaten führen, die unter anderem die den übrigen Zielsequenzen entsprechende Regionen enthalten. Die nachzuweisende Nucleinsäure kann RNA oder DNA beliebigen Ursprungs sein. Bevorzugt ist es genomische DNA.

Unter einer zu einer anderen Nucleinsäure oder Sequenz komplementären Nucleinsäure oder Sequenz wird hier eine Nucleinsäure oder Sequenz verstanden, die eine solche ununterbrochene, aufeinanderfolgende Reihe von Basen enthält, die mit einer ununterbrochenen, aufeinanderfolgenden Reihe von Basen der anderen Nucleinsäure oder Sequenz Watson-Crick oder/und Hoogsteen-Basenpaarungen bilden können. Diese Reihe ist bevorzugt länger als 10 Basen.

Unter einer zu einer anderen Nucleinsäure oder Sequenz homologen Nucleinsäure oder Sequenz wird hier eine Nucleinsäure oder Sequenz verstanden, die zu einer zu der anderen Nucleinsäure komplementären Nucleinsäure oder Sequenz komplementär ist. Dabei werden jeweils ununterbrochene Sequenzen von Basen verglichen.

Dabei ist es für den Fachmann offensichtlich, daß es neben den natürlich vorkommenden Basen auch künstliche Basen gibt, die sich hinsichtlich ihres Bindungsverhaltens zu anderen Basen nicht wesentlich von den natürlichen Basen unterscheiden. Für den Fall von Oligonucleotiden als Sonden spricht man auch von Hybridisierung. Die Bedingungen, bei denen eine optimale Hybridisierung stattfindet, sind dem Fachmann vertraut. Beispielsweise richten sich Temperatur, Sondenlänge und Salzgehalt auch nach dem GC-Gehalt der nachzuweisenden Nucleinsäure.

Das vorliegende Verfahren hat den Vorteil, daß unter sonst gleichen Bedingungen eine höhere Selektivität des Nachweises erreicht werden kann. Umgekehrt kann bei gleichbleibender Selektivität die Stringenz der Prozeßparameter (z. B. Höhe der Waschtemperatur) erniedrigt werden. Unter Selektivität wird die spezifische Sondenhybridisierung an Nucleinsäuren mit einer exakt komplementären Basensequenz unter Diskriminierung sämtlicher Nucleinsäuren mit nicht exakt komplementärer Sequenz verstanden.

Proben im Sinne der Erfindung sind Flüssigkeiten, wie Körperflüssigkeiten, Kulturmedien oder Gewebe, bzw. deren Aufbereitungsprodukte, wie Lysate, Extrakte oder Isolate, wie Serum, Plasma und davon abgeleitete Produkte.

Verfahren zur Amplifikation sind dem Fachmann prinzipiell bekannt. Beispiele sind Amplifikationen wie NASBA (EP-B-0 329 822 oder US-A-5,130,238), LCR (EP-B-0 320 208) und PCR (WO 90/01069). Bevorzugt im Sinne der Erfindung ist jedoch die Amplifikation nach dem PCR-Prinzip (EP-B-0 200 362 bzw. US-A-4,683,202). Hierbei werden mindestens 2 Primer eingesetzt, die bezüglich ihrer Sequenz so ausgewählt werden, daß einer mit einem Strang der nachzuweisenden Nucleinsäure so hybridisieren kann, daß sich mit ihm als Templat nach Einwirkung einer Polymerase und den weiteren hierfür benötigten Reagenzien, wie Puffer, Mononucleosidtriphosphaten etc. unter Verwendung einer downstream vom 3'-Ende des Primers gelegenen Nucleinsäurestücks als Templat ein Verlängerungsprodukt bildet, das wiederum als Templat für die Hybridisierung mit dem und Verlängerung des anderen Primers dienen kann. Je selektiver die Primer an die Nucleinsäure bzw. deren Gegenstrang binden, desto selektiver wird schon die Amplifikation. Durch mehrfache Ausführung der Reaktionsschritte Hybridisierung eines Primers mit seinem Templat, Verlängerung des Primers und Trennung des Verlängerungsproduktes von seinem Templat ist es möglich, eine Vielzahl von Kopien des zwischen den äußeren Enden der Primer gelegenen Nucleinsäurestückes, welches die Zielsequenz(en) enthält, herzustellen.

Unter einem forward-Primer wird ein Primer verstanden, der mit einer Sequenz des nachzuweisenden Nucleinsäurestranges hybridisieren kann bzw. hybridisiert. Der Strang kann ausgewählt werden aus einem der beiden Stränge eines Doppelstranges, kann aber auch eine selbst schon einzelsträngige Nucleinsäure, z. B. RNA, bevorzugt mRNA oder rRNA, sein. Der reverse-Primer ist ein Primer, der mit dem aus dem forward-Primer gebildeten Verlängerungsprodukt hybridisieren kann bzw. hybridisiert.

Bei dem forward-Primer handelt es sich bevorzugt um einen Primer, der mit dem anti-sense-Strang der nachzuweisenden Nucleinsäure hybridisieren kann, der reverse-Primer mit dem sense-Strang. Aus dem forward-Primer bildet sich somit ein sense-Verlängerungsprodukt (sense-Tochterstrang), FIG 1.

Neben einer auf die Primerbindungsstelle gerichteten Sequenz können die Primer auch weitere Sequenzen, die nicht direkt mit der nachzuweisenden Nucleinsäure hybridisieren können, enthalten, z. B. oligo T-Enden, die bevorzugt am 5'-Ende als Abstandshalter zu einer Markierung dienen. Auch die Fangsonden können so modifiziert sein.

Übliche Primer haben eine Länge von zwischen 12 und 30 Nucleotiden/Basen. Diese sind bei der vorliegenden Erfindung beide einbezogen in den darauffolgenden Nachweis, welcher ein Zeichen für die Anwesenheit (qualitativer Nachweis) oder die Menge (quantitativer Nachweis) der Analytnucleinsäure ist.

Im Sinne der Erfindung ist nur einer der Primer, ausgewählt aus der Gruppe der forward- und der reverse-Primer, besonders bevorzugt der reverse, nachweisbar markiert. Unter einer Markierung wird eine chemische Gruppe verstanden, die sich in ihren Eigenschaften von denen von Nucleinsäuren detektierbar unterscheidet. Eine solche Eigenschaft kann z. B. eine Absorption bei einer bestimmten Wellenlänge, Fluoreszenz, Streuung, Reflektion oder Bindefähigkeit zu anderen Substanzen sein. Man kann sie auch in direkte und indirekte Markierungen trennen. Direkte Markierungen können ohne Zusatz weiterer Bindekomponenten aufgrund ihrer signalerzeugenden Eigenschaft nachgewiesen werden. Zu dieser Gruppe zählen beispielsweise Enzyme, wie alkalische Phosphatase oder Peroxidase (POD), die durch Verfolgung der enzymkatalysierten Umsetzung eines Farbsubstrats nachgewiesen werden können. Gruppen, die diese Bedingungen erfüllen, sind jedoch im Sinne der Erfindung nur dann bevorzugt, wenn das Substrat in der Nähe des Komplexes während der Messung verbleibt, z. B. wie bei Äquorin. Hierzu zählen jedoch auch die aufgrund ihres spektralen Verhaltens nachweisbaren, signalgebenden Gruppen, wie Fluoreszein.

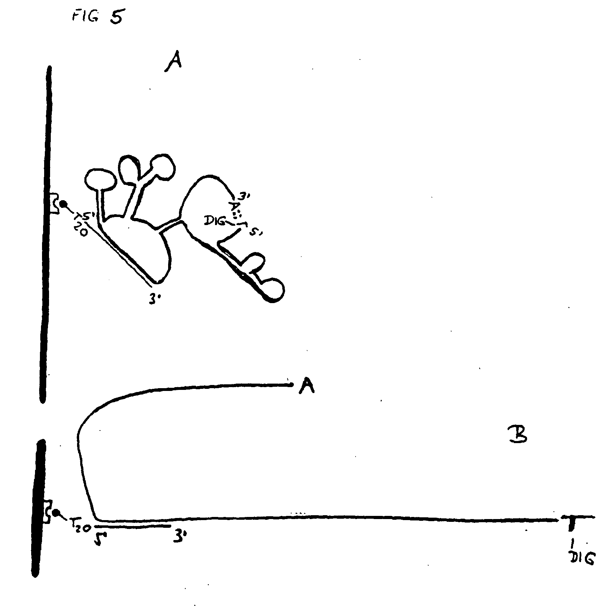

Indirekte Markierungen sind solche, die noch eine Bindung an ein weiteres Reagenz benötigen, das eine hiermit bindefähige Komponente enthält, welches dann wiederum direkt oder indirekt markiert sein kann. Zu diesen Gruppen zählen beispielsweise Haptene, wie Biotin oder Digoxigenin. Sie können durch Umsetzung mit einem Nachweisreagenz enthaltend ihre Bindepartner, z. B. Streptavidin bzw. einen Antikörper, nachgewiesen werden; dazu sind diese durch eine signalgebende oder signalvermittelnde Gruppe, z. B. durch Äquorin oder einen Fluorophor, markiert. Besonders bevorzugt als signalgebende Gruppe sind partikuläre Komponenten, z. B. Beads oder Microsphären aus Latex, die einen nachweisbaren Farbstoff beinhalten. Die Erfindung wirkt sich besonders positiv aus bei Partikeln einer großen Größe, die wegen der in FIG 5 gezeigten sterischen Situation real nicht an die Markierung (dort Digoxigenin) binden kann, wenn nicht die stranginterne Reassoziation ausgeschaltet wird. Die Markierung ist natürlich zweckmäßigerweise so an dem Primer angebracht, daß sie die Hybridisierung und Verlängerung des Primers möglichst wenig behindert. Bevorzugt ist dabei die Anbringung am 5'-Ende im Falle eines Oligonucleotides, z. B. während der chemischen Synthese, unter Verwendung eines mit der Markierungsgruppe derivatisierten Phosphoramidites. Die Markierung kann jedoch auch an einer Base angebracht sein (WO 93/05060). Im Falle von PNA wird die Markierung bevorzugt am Aminoende angebracht (WO 92/20703).

Unter einer Fangsonde wird eine Nucleinsäure bindende Einheit verstanden, die eines der durch Amplifikation hergestellten Nucleinsäurestränge (Verlängerungsprodukte) selektiv binden kann und entweder an eine Festphase gebunden werden kann (immobilisierbar) oder gebunden ist (immobilisiert). Unter direkter Bindung wird in diesem Zusammenhang die Bindung zweier Basen mit Hilfe von Wasserstoffbrücken verstanden. Die Bindung an eine Festphase kann kovalent oder nicht-kovalent sein. Kovalente Bindungen können entweder gewährleistet werden durch Phosphodiesterbindungen (EP-B-0 386 229) und Amidbindungen (EP-B-0 562 025 oder US-A-5,242,974) aber auch durch Aktivierung photosensitiver Reste (z. B. Diazogruppen) und Inkontaktbringen mit einer Lösung in eine Sonde, die eine reaktive Gruppe (z. B. PCT/EP 96/00893) enthält.

Nicht-kovalente Bindungen sind beispielsweise biospezifische Bindungen, wie enthalten in den Wechselwirkungspaaren (Strept)avidin-Biotin, Hapten-Antikörper, Vitamin-Rezeptor etc.. Bevorzugt ist die Fangsonde biotin-markiert, z. B. über eine 5'-Phosphatgruppe mit Hilfe eines Aminoalkyl-Linkers und die Festphase ist Streptavidin-überzogen. Besonders bevorzugt ist die Fangsonde jedoch schon vor Inkontaktbringen mit der Reaktionsmischung aus der Amplifikation kovalent oder über eine stabile biospezifische Bindung (z. B. (Strept)Avidin-Biotin) an die Festphase gebunden. Die Konstitution der Festphase ist für die vorliegende Erfindung nicht wesentlich. Sie sollte jedoch in der Amplifikationsmischung unlöslich sein. Besonders geeignet sind z. B. Phasen aus Kunststoffen mit oder ohne metallischen Anteil, z. B. in Form von Perlen, aber auch eines porösen Vlieses oder eines mehr oder weniger flachen, nicht-porösen Testträgers. Wesentliche Eigenschaft des Trägers ist die Darstellung eines Ortes, an welchen die Fangsonden gebunden sind oder werden können, und woran dann im Falle der Anwesenheit, die nachzuweisende Nucleinsäure binden kann. Bevorzugt wird nicht gleichzeitig zu einer Fangsonde mit einer bestimmten Sequenz auch eine Fangsonde eingesetzt, die zu 100 % komplementär zu der Fangsonde ist.

Eine besondere Ausführungsform der Erfindung ist auf den potentiellen (Mehrfach)Nachweis einer Mehrzahl von Nucleinsäuren unterschiedlicher Sequenz gerichtet. Dies kann dadurch geschehen, daß für jede nachzuweisende Nucleinsäure bzw. Sequenz eine gerade hierfür selektive Fangsonde eingesetzt wird. Die Fangsonden können zwar an unterschiedliche Festphasen gebunden sein, besonders bevorzugt an unterschiedlichen Stellen, besonders bevorzugt an definierte und meßtechnisch unterscheidbare geometrische Orte auf derselben Festphase gebunden sein, bevorzugt nach Art der Nucleinsäurearrays, wie beispielsweise beschrieben in WO 92/10588. Hierbei können bestimmte geometrische Muster gewählt werden, z. B. rechteckige, hexagonale oder kreuzförmige Matrices, die die Auswertung erleichtern. Damit können ganze Cluster von Zielsequenzen bezüglich ihrer Anwesenheit bzw. Abwesenheit in einer Probe untersucht werden. Die vorliegende Erfindung benutzt die Bildung eines Bindekomplexes (Hybrides), welches sowohl das Verlängerungsprodukt des reverse- als auch des forward-Primers als auch die Fangsonde enthält. Hierbei wird im Folgenden von dem Verlängerungsprodukt als forward-Verlängerungsprodukt gesprochen, welches durch Verlängerung des forward-Primers gebildet wurde. In den aus dem Stand der Technik bekannten Verfahren wurden nicht die Bedingungen eingehalten, die für die meßbare Bildung eines solchen Hybrides erforderlich sind. Generell ist die Bildung eines solchen Komplexes bevorzugt, wenn der Fangsonde ausreichend Gelegenheit zur Hybridisierung mit einem Strang des Amplifikates gegeben wird, bevor dieser mit seinem (Amplifikat-)Gegenstrang unter Bildung eines (Re-)assoziationsproduktes hybridisieren kann. Geschieht dies nicht, kann die Fangsonde gerade bei zu starker stranginterner Hybridbildung neigender nachzuweisender Nucleinsäuren nicht mehr mit dem gewünschten Verlängerungsprodukt hybridisieren und ein Nachweis ist erschwert oder unmöglich gemacht, da die Signalstärke dann recht gering ist.

Dies kann folgendermaßen erreicht werden; eine geeignete Menge an Hybridisierungslösung sowie ein Aliquot aus dem Reaktionsgemisch aus der Amplifikation, in welchem als Doppelstränge vorliegende Verlängerungsprodukte durch Basendenaturierung einzelsträngig gemacht wurden, werden jeweils getrennt oder/und nacheinander in eine Pipette, bevorzugt in dieselbe Pipette, physikalisch voneinander getrennt (so daß eine Vermischung in der Pipette im wesentlichen ausgeschlossen ist) aufgenommen und dann in ein Gefäß ausgestoßen, welches die Festphase enthält.

In einer bevorzugten Ausführungsform wird zuerst die Hybridisierungslösung, dann eine Luftblase und dann das Reaktionsgemisch in eine Pipette oder eine Nadel aufgesaugt, so daß sich die beiden Flüssigkeiten möglichst nicht vermischen. Dann werden die beiden Flüssigkeiten schnell, d. h. innerhalb eines Zeitraumes von weniger als 5 sec., bevorzugt weniger als 1 sec. in ein Gefäß ausgestoßen, welche die Fangsonde enthält. Eine rasche Vermischung wird insbesondere erreicht durch die Schnelligkeit des Ausstoßes und gewünschtenfalls durch wiederholtes Aufnehmen und Ausstoßen der Flüssigkeit in die und aus der Pipette. Die Schnelligkeit des Ausstoßes muß hierbei der Geometrie des Reaktionsgefäßes angepaßt sein, z. B. um Verspritzungen zu vermeiden. Durch eine rasche Vermischung ist gewährleistet, daß die Hybridisierung eines Stranges des Verlängerungsproduktes mit einer Fangsonde praktisch gleichzeitig vonstatten gehen kann, wie die Hybridisierung mit dem Gegenstrang oder stranginterne Hybridisierung. Der Startzeitpunkt für die Hybridisierung wird mit t₀ bezeichnet und ist der Zeitpunkt, bei dem Reaktionsbedingungen eingestellt sind, die eine Hybridisierung erlauben.

Bezüglich der Inkubations-Temperatur ist es bevorzugt, eine solche zu wählen, bei der die Fangsonde mit dem sense-Strang hybridisieren kann, bevorzugt ≥ 5°C unterhalb des Schmelzpunktes dieses Hybrids und ≥2 °C oberhalb des Gefrierpunktes der Mischung.

Die Inkubation der Sonde(n) mit dem Reaktionsgemisch findet bevorzugt zwischen 45 und 180 min statt. Anschließend wird der nicht an die Festphase gebundene Anteil von Primern (insbesondere des markierten Primers) sowie von markierten Amplifikationsprodukten von der Festphase abgetrennt, so daß nicht selektiv gebundene, d. h. überschüssige, Markierung, die Bestimmung des selektiv gebundenen Anteils nicht wesentlich stört. Dies kann durch Abpipettieren der Flüssigkeit, gewünschtenfalls unterstützt durch einen oder mehrere Waschschritte, geschehen.

In einer bevorzugten Ausführungsform der Erfindung werden die Hybridisierungsbedingungen während der Inkubtion der Fangsonde mit den Amplifikaten relativ unstringent gewählt, d. h. so, daß noch keine selektive Hybridisierung stattfindet, sondern relativ viel Nucleinsäure relativ unselektiv gebunden wird. In einem oder mehreren der nachfolgenden Waschschritte hingegen werden relativ stringente Hybridisierungsbedingungen gewählt. Dabei lösen sich die Hybride wieder auf, in denen die Fangsonde relativ wenig komplementär zur Zielsequenz ist, während die strenger komplementären Hybride gebunden bleiben.

Dazu wird der Waschpuffer bevorzugt folgendermaßen ausgesucht. Er enthält 2 bis 5 mol/l TMAC, 0.1 - 5 mmol/l eines Chelatbildners (z. B. EDTA), 0.1 bis 5 Gew.% eines bevorzugt anionischen Detergenz sowie 1 bis 100 mM einer organischen Pufferbasis.

Das vorliegende Verfahren eignet sich besonders für Nachweise von Mutationen, insbesondere komplexen Musters, z. B. bei Resistenzen von Organismen, wie gegen Antibiotika oder Polymorphismen, wie bei p53. Es ist dadurch gekennzeichnet, daß es besonders selektiv ist, d. h. besonders gut zwischen sehr ähnlichen Nucleinsäuresequenzen unterscheiden bzw. diskriminieren kann.

In einer bevorzugten Ausführungsform wird die Probe, z. B. ein Serum, mit Hilfe von Reagenzien so behandelt, daß die nachzuweisenden Nucleinsäuren in für die Hybridisierung zugänglicher Form vorliegen. Dies kann beispielsweise geschehen durch Zusatz von Reagenzien, die Zellwände von Organismen, z. B. Viren oder Bakterien, lysieren. Falls gewünscht können die Nucleinsäuren vorgereinigt werden, z. B. durch Immobilisierung an einer Festphase, wie an Glaspartikeln unter Zusatz von chaotropen Salzen. Solche Vorreinigungen sind beispielsweise beschrieben in EP-A-0 389 063 oder US-A-5,234,809. Die nucleinsäurehaltige Lösung wird nun einer Nucleinsäureamplifikation durch PCR unterzogen, wobei der reverse-Primer mit einer nachweisbaren Gruppe markiert ist, der forward-Primer nicht. Der zwischen den Primern liegende Bereich der nachzuweisenden Nucleinsäure ist derjenige, der die Zielsequenz enthält. Die amplifikathaltige Lösung wird gleichzeitig mit einer Hybridisierungslösung in ein flaches Reaktionsgefäß gegeben, an dessen Oberfläche in diskreten Bereichen Fangsonden unterschiedlicher Sequenz gebunden sind, wobei die Fangsonden komplementär zu jeweils einer Zielsequenz auf dem unmarkierten sense-Strang einer der nachzuweisenden Nucleinsäure sind. Sind umgekehrt die Fangsonden antiparallelkomplementär zum anti-sense Strang konzipiert, wird entsprechend der forward-Primer in markierter Form eingesetzt.

Dabei bilden sich erfindungsgemäß die gewünschten Hybride aus der Fangsonde und den beiden Strängen, sofern die nachzuweisende Nucleinsäure in der ursprünglichen Probe enthalten war. Die Bildung der Hybride kann über die eingebaute Markierung in den Amplifikaten nachgewiesen werden, z. B. direkt über ein (Fluoreszenz-)Mikroskop. Bei indirekten Markierungen, z. B. durch ein Hapten, wird zunächst mit einem markierten Antikörper gegen das Hapten umgesetzt, so daß der Antikörper und somit die (zweite) Markierung an die (erste) Markierung bindet. Danach ist die Messung der (zweiten) Markierung möglich.

Die Auswertung der Messung geschieht dahingehend, daß festgestellt wird, an welchen Stellen eine Markierung gemessen wird. Die zu der dort gebundenen (bekannten) Sequenz der Fangsonde komplementäre Sequenz ist dann in der Probe vorhanden. Üblicherweise wird bei der Entscheidung, ob das gemessene Signal als positiv zu werten ist, ein Schwellenwert definiert, oberhalb dessen angenommen wird, daß die Nucleinsäure vorhanden war. Da die Meßsignale für unterschiedliche Sequenzen und Ähnlichkeiten schwanken können, die Meßempfindlichkeit jedoch meist so hoch ist, daß auch Hintergrundsignale als Signal gewertet werden könnte, ist die durch die vorliegende Erfindung erreichbare höhere Diskriminierung außerordentlich wichtig, um falsche Auswertungen zu vermeiden. Es kann sehr viel besser zwischen den Signalen bei Anwesenheit und Abwesenheit der Zielsequenz differenziert werden.

Bei solchen Vielfachtests ist es einerseits möglich, verschiedenste Analyten, z. B. Viren, wie HBV, HGV, HCV und HIV, miteinander unter Verwendung verschiedener Primerpaare zu detektieren, oder Teilsequenzen einer bestimmten Nucleinsäure, z. B. Polymorphismen auf menschlichen Genomen, nachzuweisen, jedoch ist es auch möglich, Sequenzen nach dem "Sequencing-by-hybridization"-(SBH)-Verfahren zu sequenzieren, entweder de-novo (bislang unbekannte Sequenzen) oder auch zum Auffinden von Abweichungen von einer normalen Sequenz. Ein SBH-Verfahren ist beispielsweise beschrieben in Khrapko et al., FEBS Letters 250, 118 - 122 (1989) oder Khrapko et al., J. DNA Sequencing and Mapping 1, 375 - 388 (1991). Dazu werden üblicherweise Fangsonden ungefähr gleicher Länge verwendet, deren Sequenzen so überlappen, daß sie am einen Ende kürzer und am anderen Ende länger sind als die nächstähnliche Sequenz. Umgekehrt gilt für jede weitere Sequenz, daß sie in unveränderter Richtung am einen Ende länger, aber am anderen kürzer ist. Der so gebildete Versatz beträgt, je nach Bedürfnissen, zwischen 1 und 5 Nucleotiden. Die Sequenz der zu sequenzierenden Nucleinsäuren kann durch Zusammensetzen der erhaltenen Hybridisierungsinformationen ermittelt werden.

Für die Mutationsanalyse können allel- oder mutationsspezifische Fangsonden eingesetzt werden.

Ebenfalls Gegenstand ist daher ein Verfahren zum selektiven Nachweis einer Nucleinsäure enthaltend die Schritte Amplifikation der Nucleinsäure oder eines Teils davon mit Hilfe zweier Primer, von denen einer mit einem Strang der nachzuweisenden Nucleinsäure und der andere mit einem hierzu komplementären Strang hybridisieren kann und von denen nur einer eine nachweisbare Markierung gebunden enthält, unter Bildung jeweils eines Verlängerungsproduktes dieser Primer in einer Reaktionsmischung, Bindung einer Fangsonde an eines dieser Verlängerungsprodukte unter Ausbildung eines Hybridisierungskomplexes, der die Fangsonde und mindestens dieses Verlängerungsprodukt enthält, Abtrennung des an die Fangsonde gebundenen Verlängerungsproduktes von nicht gebundenen Anteilen der Reaktionsmischung, und Bestimmung der an die Fangsonde gebundenen nachweisbaren Markierung, dadurch gekennzeichnet, daß die Fangsonde so gewählt wird, daß sie mit dem Strang des Verlängerungsproduktes binden kann, der auch mit dem durch Verlängerung des markierten Primers gebildeten Verlängerungsprodukt hybridisieren kann.

Ebenfalls Gegenstand ist ein Reagenzkit zum Nachweis von Nucleinsäuren wie in Anspruch 4 definiert.

Ebenfalls Gegenstand der Erfindung ist die Verwendung eines Reagenzienkits wie in Anspruch 5 definiert.

Die vorliegende Erfindung wird durch die folgenden Beispiele erläutert:

### Beispiele

### Beispiel 1

### Nachweis von Mycobakterium tuberculosum

### A. Probenvorbereitung und Amplifikation

In E. coli klonierte DNA-Plasmidstandards, die das Gen für die β-Untereinheit der RNA-Polymerase (rpo-ß) von Mycobacterium tuberculosis enthalten (Wildtyp WT und Mutanten MX), wurden nach Zelllyse isoliert und über Qiagen-Säulchen gereinigt. Das für Rifampicin-Resistenz relevante rpo-β-Gensegment wurde via PCR amplifiziert, wobei wahlweise der forward- oder der reverse-Primer am 5'-Ende eine DIG-Markierung trug. Die Primer-Oligonucleotide wurden gemäß Standard-Phosphoramiditchemie synthetisiert (Caruthers, M.H., Barone A.D., Beaucage, S.L., Dodds, D.R., Fisher, E.F., McBride, L.J., Matteucci, M., Stabinsky, Z., Tang, J.Y., Chemical synthesis of deoxyribonucleotides, Methods in Enzymology 154, 287-313 (1987)) und ggfs. 5'-terminal funktionalisiert via Einbau von [N-Trifluoroacetamido-(3-oxa)pentyl-N,N-diisopropyl-methyl-phophoramidit (Firma Boehringer Mannheim GmbH (BM), Best. Nr. 1480863), nachfolgender basische Abspaltung der Trifluoracetyl-Schutzgruppe mit wässrigem Ammoniak und Umsetzung mit Digoxigenin-3-O-methylcarbonyl-6-amidocaproyl-NHS-ester (Firma BM, Best Nr. 1333054).

Das PCR-Temperaturprofil, gefahren auf einem Perkin-Elmer GeneAmp PCR System 9600 mit Einsatz von 1 µl gereinigtem Zellextrakt pro 100 µl PCR Reaktionslösung, lautete:
Initiation 3' 94°C; 10 Zyklen 15" 95°C / 30" 68°C / 30" 72°C; 20 Zyklen 15" 94°C / 30" 68°C / 30" + zusätzlich 20" mit jedem neuen Zyklus bei 72°C; 5' 70°C. Äquilibrierung ≥ 30' 4°C
PCR Puffer: 10 mM Tris/HCl, 1.5 mM MgCl₂, 50 mM KCl, , pH 8.3, Primer (jeweils forward und reverse, siehe unten) Polymerase und dNTPs gemäß PCR Core Kit (BM, Kat. Nr. 1578553).
PCR-Primer (R = reverse; F = forward):
Primer 55 F:5'-TCG CCG CGA TCA AGG AGT-3' (SEQ.ID.NO. 1)
Primer 55 R:5'-TGC ACG TCG CGG ACC TCC A-3' (SEQ.ID.N0.2)
Primer 56 F : 5'- DIG-TCG CCG CGA TCA AGG AGT-3' (SEQ.ID.NO.3)
Primer 56 R : 5'- DIG-TGC ACG TCG CGG ACC TCC A-3' (SEQ.ID.NO.4)
MTB rpo-β-Amplicon (sense-Strang; 157 nt):

Amplifizierte Plasmidstandards, Wildtyp (WT) oder Mutanten (MX), wurden nach Kontrolle über Gelelektrophorese und Ethidiumbromid-Anfärbung aliquotiert und bis zur Benutzung tiefgefroren aufbewahrt.

### B) Denaturierung der Amplifikate

Es werden zunächst 5 µl Lösung des DIG-markierten Amplifikates und 5 µl einer stark basischen Denaturierungslösung (50 mM NaOH, 2 mM EDTA) in einem inerten Reaktionsgefäß (z. B. von der Fa. Eppendorf) gemischt und ≥ 10 min bei RT inkubiert.

### C) Herstellung des Disposables

Fangsonden, komplementär antiparallel zum sense-Amplicon-Strang, wurden ebenfalls gemäß Standard-Phosphoramiditchemie als 18-mere mit 5'-terminaler Biotinylierung synthetisiert. Die Biotin-Gruppe wurde direkt bei der Synthese eingebaut mittels eines Biotinoyl-6-amidohexyl- N,N -diisopropyl-ß-cyanoethyl-phosphoramidits, bei dem der reaktive imidazolyl-Stickstoff Dimethoxytrityl-geschützt ist ( Firma Perkin-Elmer ABI, Nr. 401396).

c = komplementär zum sense-Strang; S = sensitiv (auf Rifampicin-Behandlung (= den Wildtyp erfassende Sonden); R = resistent gegenüber Rifampicin-Behandlung (= diverse Mutanten erfassende Sonden)
cS1-18/2-BIO 5'-BIO-T₂₀-ATT GGC TCA GCT GGC TGG-3' (Tab. 4) SEQ.ID.NO.6
cR1a-18-BIO 5'-BIO-T₂₀-TTG GCT CGG CTG GCT GGT-3' (Tab. 4) SEQ.ID.NO.7
cS2-18-BIO 5'-BIO-T₂₀-GTT GTT CTG GTC CAT GAA-3' (Tab. 4) SEQ.ID.NO.8
cR2-18-BIO 5 '-BIO-T₂₀-GTT GTT CTG GAG CAT GAA-3' (Tab. 4) SEQ.ID.NO.9
cS3-18/1-BIO 5'-BIO-T₂₀-CAA CCC CGA CAG CGG GTT-3' (Tab.1-3) SEQ.ID.NO. 10
cS3-18/2-BIO 5'-BIO-T₂₀-TCA ACC CCG ACA GCG GGT-3' (Tab.4) SEQ.ID.NO.11
cS4-18-BIO 5'-BIO-T₂₀-TCG GCG CTT GTG GGT CAA-3' SEQ.ID.NO.12
cR4a-18-BIO 5'-BIO-T₂₀- TCG GCG CTT GTA GGT CAA-3' SEQ.ID.NO 13
cR4b-18-BIO 5'-BIO-T₂₀- TCG GCG CTT GTC GGT CAA-3' SEQ.ID.NO 14
cdS4-18-BIO 5'-BIO-T₂₀- TCG GCG CTT GCG GGT CAA-3' (Tab. 4) SEQ.ID.NO.15
cS5-18-BIO 5'-BIO-T₂₀-CCC CAG CGC CGA CAG TCG-3' SEQ.ID.NO 16
cR5-18-BIO 5'-BIO-T₂₀-CCC CAG CGC CAA CAG TCG-3' (Tab. 4) SEQ.ID.NO. 17

- BIO =: Monobiotinylierung via on-line-Einbau von Biotinoyl-6-amidohexyl-phosphor-amidit
- Y =: on-line-Einbau von 3-Trifluoacetamido-1,2-propandiol-phosphoramidit, nach-folgend basische Entblockung, Verlängerung mit 6-Aminocapronsäure NHS-Ester und Markierung über Umsetzung mit DIG-3-O-carboxymethyl-NHS-ester (basen-stabil) (siehe oben).

Als Disposables dienten mit schwarzem Kohlenstoffpigment eingefärbte Polystyrol-Disposables (DE-19707204.6) mit einer Vertiefung der Maße 0.7 cm (rund, Durchmesser) x 0.15 cm (tief), die mit einer thermoRSA-Biotin / Streptavidin-Schicht aktiviert wurden (PCT/EP 89/00195). Biotin-funktionalisierte Fangsonden wurden darauf durch einen Inkjet Printing-Prozeß analog EP-A-0 268 237 immobilisiert. Jede Düse des Druckkopfes wurde jeweils mit einer Fangsonden-Lösung anderer Spezifität befüllt, so kleine, voneinander isolierte, kreisrunde Reaktionsfelder mit einem Durchmesser von ca. 100 µm in geometrischen Mustern auf den Testträger gedruckt, getrocknet und damit ein Array verschiedener Fangsonden generiert (C2a-Festphase).

Drucklösung (inkjet printing solution): 5 mM Mes/5 mM Tris-HCl, pH 7.4, 1% (w/v) Saccharose, 0.5 mg/ml RSA-Res (dialysiert), Sonden-Konzentration 1 µM.

### D) Sondenhybridisierung

Danach wird z. B. mit einem Hamilton MicroLab-Dispenser nacheinander, und durch eine Luftblase getrennt, 40 µl Hybridisierungs- (= Neutralisations-)Puffer und 10 µl denaturiertes PCR-Amplifikat aufgezogen, und anschließend druckvoll in einem Schritt ins Disposable dispensiert, so daß eine rasche Vermischung eintritt und ein identisches t₀ für alle Hybridisierungsereignisse vorliegt. Die Mischung wurde für 90 min bei Raumtemperatur (Tabellen 1 bis 3) inkubiert, bzw. 37 °C (Tabelle 4), mit (Tabellen 1 bis 3) bzw. ohne (Tabelle 4) Schütteln (E. Bühler Swip, 250 rpm, linear).

### Hybridisierungspuffer:

10 mM Tris/HCl, 4 M TMAC (Tetramethylammonium-chlorid), 1 mM EDTA, 0.1% Tween-20 [in Bsp.4: Zwittergent 3-12] (w/v), 0.013% Oxypyrion, 0.01 % Methylisothiazolon, pH 6,3.

### E) Waschschritt

Zuerst 20 sec b/f-Trennung bei 59 (61)°C (eingestellte Solltemperatur an der manuellen DNA-Waschvorrichtung) bzw. 60°C (Isttemperatur am semiautomatischen Inkubator/Schüttler/Waschmodul) mit Waschlösung 1 (Flußrate 14.4 bzw 12 ml/min); sofort hinterher 20 sec mit salzarmem Waschpuffer bei RT nachwaschen (Waschlösung 2, Flußrate 12 ml/min).

Waschlösung 1 (für Stringenz-Waschschritt nach Hybridisierung):
10 mM Tris/HCl, 4 M TMAC, 1 mM EDTA, 0.1% Tween-20 [in Tab.4: Zwittergent 3-12] (w/v), 0.013 % Oxypyrion, 0.01 % Methylisothiazolon, pH 8.0.

Waschlösung 2 (zum Nachwaschen bei der bound/free-Trennung nach Hybridisierun, sowie zur bound/free-Trennung nach Konjugatreaktion):
15 mM NaCl / 1.5 mM Na₃-Citrat / 0.1 % SDS-Lösung, pH 7.0

### F) Konjugatbindung

30 µl Konjugatsuspension (anti-DIG-funktionalisierte Fluorobeads in stabiler Suspension 0.01 Gew. %) wurden in die Mulde pipettiert und die Mischung 30 min bei Raumtemperatur (60 min, 37 °C in Tabelle 3) inkubiert. Für die abschließende DIG : Anti-DIG-Immunreaktion mit Schütteln (E. Bühler Swip, 250 rpm, linear) (Tabellen 1 bis 3) bzw. ohne (Tabelle 4).

Anschließend wurde 8 sec mit salzarmem Waschpuffer (Waschlösung 2) bei RT und einer Flußrate von 12 ml/min gewaschen.

### Konjugatsuspension:

MAK<DIG>(IgG)-110 nm COOH-Latex-Fluorobead-Suspension (hergestellt aus BM-Biochemicals Beads, Kat. Nr. 1742582, verwendet gemäß US-A-5,516,635) 0.1 % Gew.-solids wird hergestellt durch Anlösen eines Fläschchens Konjugat-Lyophilisat mit 200 µl Aqua bidest. Zubereitung von Tagesbedärfen durch 1:10-Verdünnung auf 0.01 % solids in Konjugatpuffer.

### Konjugatpuffer:

50 mM Tris/HCl, pH 8.5, 150 mM NaCl, 0.5 % RPLA-IV, 10 µg/ml M-IgG-1(poly-Fab, BM, Biochemicals for the Diagnostic Industry, Kat. Nr. 1368388), 10 µg/ml M-IgG-2a(poly-Fab, BM, Biochemicals for the Diagnostic Industry, Kat. Nr. 1866729), 0.05 % (w/v) Tween-20, 0.095 % NaN₃.

### G) Messung der gebundenen Fluoreszenz und Auswertung

Detektion geschah mit einem Mikroskop/CCD-Kamera-Aufbau von der Firma. Leica (Heidelberg, BRD).

In den Tabellen 1-3 sind Meßergebnisse zusammengestellt, die mit dem anfänglichen 5X (5 Sonden) Array bei vergleichender Bewertung von F- (= via forward-Primer markierte sense-Stränge) und R- (= via reverse-Primer markierte anti-sense-Stränge) Amplicons unter manueller Testführung erbracht wurden. Variiert wurden die Waschtemperatur im Stringenzwaschschritt nach Hybridisierung sowie Inkubationstemperatur bzw. -zeit für die Konjugatreaktion.

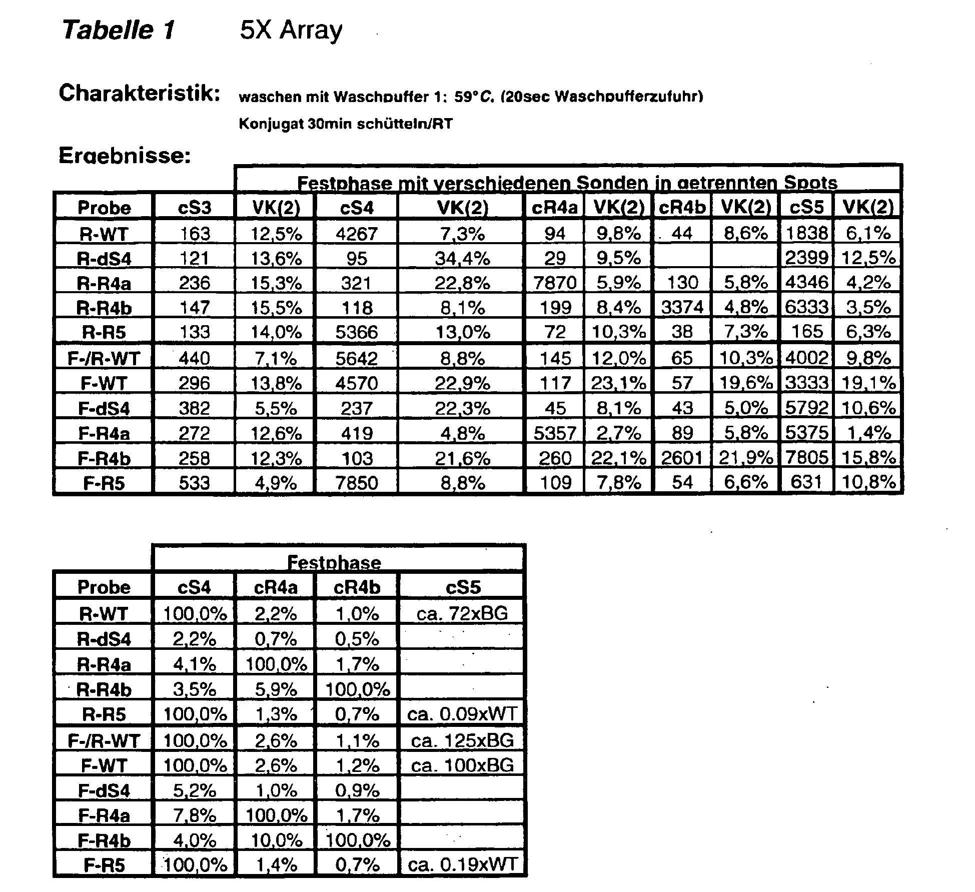

Die grau hinterlegten Felder kennzeichnen ein Hybridisierungsereignis mit einer exakt komplementären Sonde.

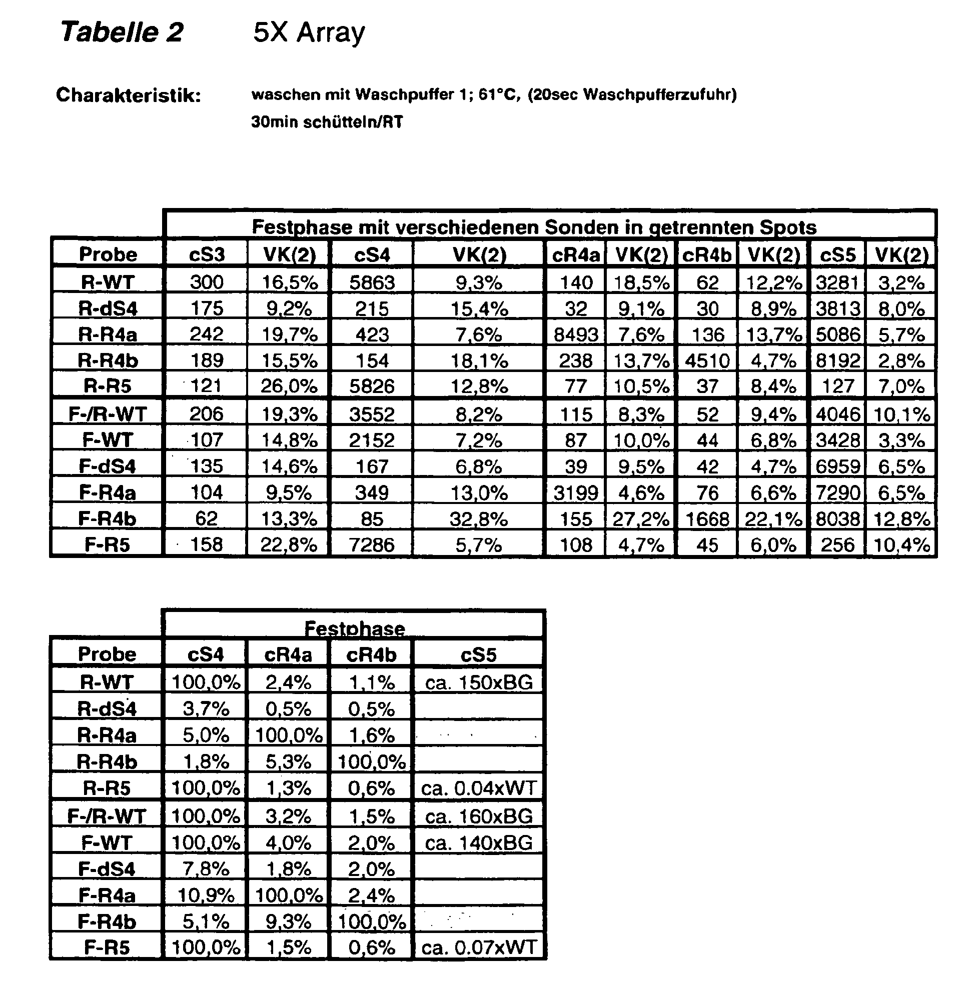

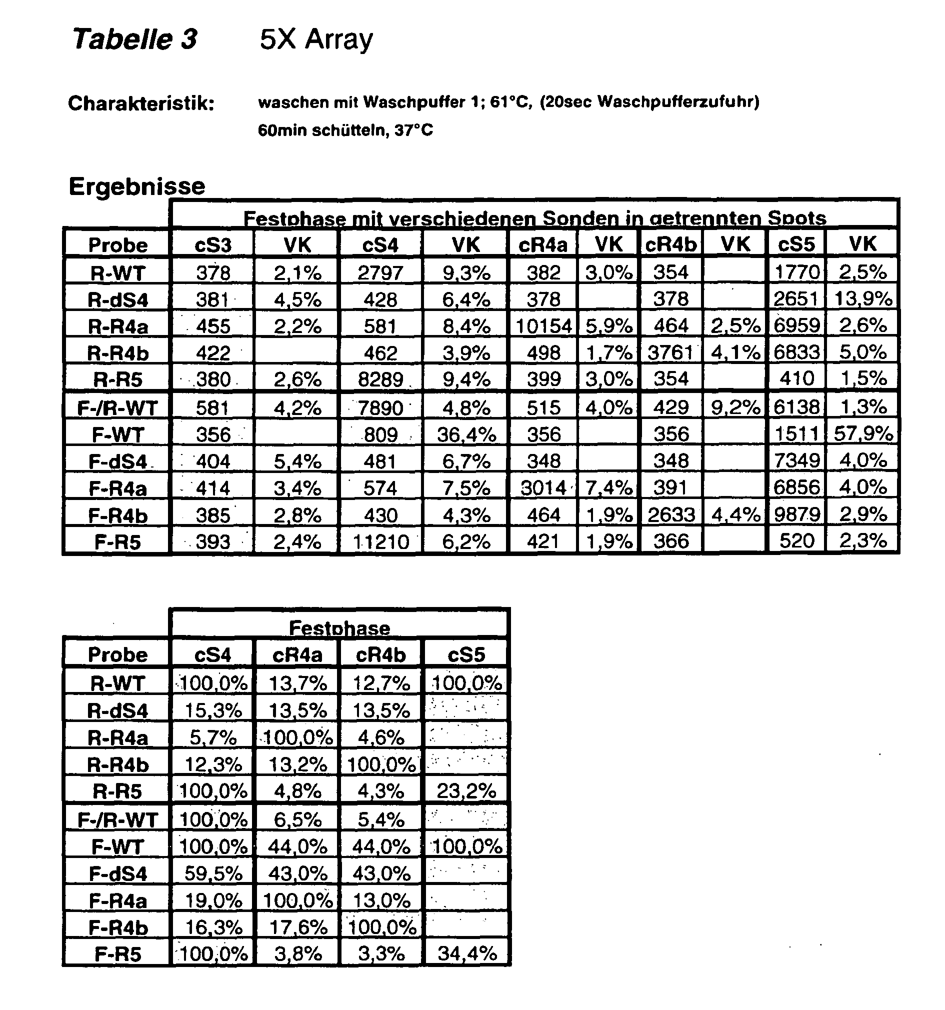

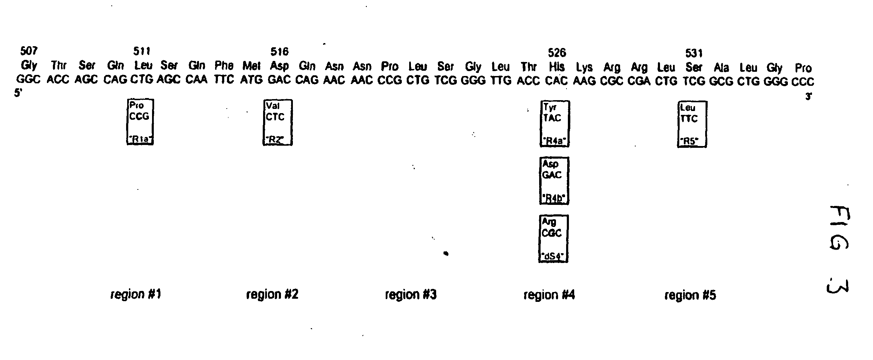

Tabelle 4 zeigt dasselbe für das erweiterte 12X (12 Sonden) Array unter den Bedingungen 90 min Hybridisierung bei 37°C, 30 min Konjugatreaktion bei RT, Probenwaschschritt bei 60°C für 20 sec, durchgeführt am semi-automatischen DNA Inkubator/Schüttler/Waschmodul. Zu diesem Zeitpunkt war der Test für die neu hinzugekommenen Regionen 1 und 2 noch nicht optimiert, so daß primär die Regionen 4 und 5 (wie beim kleineren anfänglichen Array) heranzuziehen sind. Die Lage der Regionen ist in FIG 3 angegeben.

**Tabelle 4 12X Array**

| **R- Amplicon** | | | | | | | | |
|---|---|---|---|---|---|---|---|---|
| | | **Festphase mit verschiedenen Sonden in getrennten Spots** | | | | | | |
| **Probe Nr** | **Name** | **cS3** | **cS4** | **cR4a** | **cR4b** | **cdS4** | **cS5** | **cR5** |
| 3 | WT 3.11 | | 100,0% | 2,1 % | 0.5% | 7,1% | 100.0% | 0,8% |
| 4 | WT 3.11 | | 100,0% | 2,4% | 0,3% | 5,2% | 100,0% | 1,2% |
| 5 | R1a | | 100,0% | 0.5% | 0,2% | 1,2% | 100,0% | 2,1 % |
| 6 | R1a | | 100,0% | 0,4% | 0,2% | 2,6% | 100,0% | 2,8% |
| 7 | R2 | | 100,0% | 0,4% | 0,1% | 0,4% | 100.0% | 1,1% |
| 8 | R2 | | 100,0% | 0,4% | 0,1% | 0,8% | 100,0% | 1,5% |
| 9 | R4a | | 12,9% | 100.0% | 1.5% | 2,0% | 100,0% | 0.9% |
| 10 | R4a | | 13,1% | 100,0% | 3,3% | 5,3% | 100,0% | 3,1 % |
| 11 | R4b | | 14,3% | 7,9% | 100,0.% | 9,6% | 100,0% | 2,7% |
| 12 | R4b | | 7,4% | 4,2% | 100,0% | 5,2% | 100,0% | 0,8% |
| 13 | dS4 | | 19,1% | 0,5% | 0,2% | 100,0% | 100,0% | 1,3% |
| 14 | dS4 | | 15,5% | 0,4% | 0,1% | 100,0% | 100,0% | 0,6% |
| 15 | R5 | | 100,0% | 2,5% | 2,3% | 14,2% | 0,4% | 100,0% |
| 16 | R5 | | 100,0% | 1,5% | 0,7% | 9,0% | 1,4% | 100,0% |
| 17 | WT **F/R-Amp.** | | 100,0% | 6,8% | 1,2% | 10,4% | 100.0% | 3,2% |
| 18 | WT **F/R-Amp.** | | 100,0% | 4,9% | 1,1% | 22,1% | 100,0% | 1,8% |
| 19 | WT 3.11. | | 100,0% | 3,0% | 0,3% | 8,2% | 100,0% | 1,0% |
| 20 | WT 3.11 | | 100.0% | 3,0% | 0,6% | 20,4% | 100,0% | 0,6% |
| | | | | | | | | |

| **F- Amplicons** | | | | | | | | |
|---|---|---|---|---|---|---|---|---|
| | | **Festphase** | | | | | | |
| **Probe Nr** | **Name** | **cS3** | **cS4** | **cR4a** | **cR4b** | **cdS4** | **cS5** | **cR5** |
| 3 | WT 3.11 | | 100,0% | 4,6% | 1,0% | 20,3% | 100,0% | 3,4% |
| 4 | WT 3.11 | | 100,0% | 7,6% | 2,6% | 18,2% | 100,0% | 2,8% |
| 5 | R1a | | 100,0% | 0,5% | 0,2% | 1,7% | 100,0% | 1,5% |
| 6 | R1a | | 100,0% | 0,8% | 0,5% | 0,9% | 100,0% | 3,4% |
| 7 | R2 | | 100,0% | 1,7% | 0.1 % | 5,3% | 100.0% | 3,6% |
| 8 | R2 | | 100,0% | 3,4% | 0,6% | 2,6% | 100,0% | 5,8% |
| 9 | R4a | | 53,6% | 100,0% | 2,4% | 1,4% | 100.0% | 4,1% |
| 10 | R4a | | 90,4% | 100,0% | 1,2% | 5,8% | 100,0% | 2,4% |
| 11 | R4b | | 29,3% | 15,5% | 100.0% | 15,3% | 100,0% | 8,3% |
| 12 | R4b | | 54,7% | 25,9% | 100,0% | 16,6% | 100,0% | 8,9% |
| 13 | dS4 | | 50,4% | 1.8% | 0,7% | 100,0% | 100,0% | 1,8% |
| 14 | dS4 | | 51,0% | 2,3% | 0,5% | 100,0% | 100,0% | 0,3% |
| 15 | R5 | | 100.0% | 10,9% | 12.3% | 5,6% | 6.2% | 100,0% |
| 16 | R5 | | 100,0% | 1,9% | 0,4% | 3,6% | 2,4% | 100,0% |
| 17 | WT F/R-Amp. | | 100,0% | 5,8% | 2.8% | 17,8% | 100.0% | 1.8% |
| 18 | WT F/R-Amp. | | 100,0% | 5,5% | 0,7% | 24,6% | 100,0% | 2,3% |
| 19 | WT 3.11. | | 100,0% | 6,4% | 2,6% | 6,4% | 100,0% | 3.3% |
| 20 | WT 3.11 | | 100,0% | 4,5% | 0,6% | 21,9% | 100,0% | 1,1 % |

In allen Fällen wird deutlich, daß bei Verwendung von R-Amplicons, d. h. indirekter Nachweis über den Strangreassoziationskomplex, überraschend eine signifikant bessere Diskriminierung erreichbar war als mit direktem Nachweis von sondengebundenen F-Amplicons.

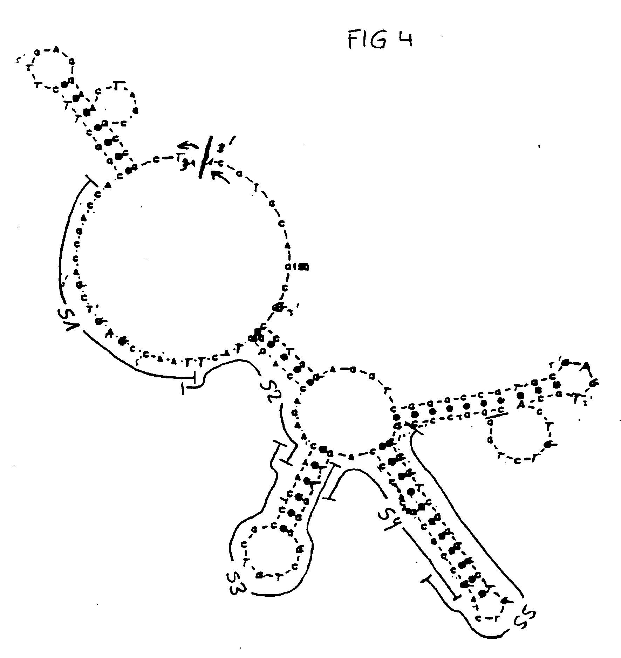

Dieser überraschende Effekt kommt besonders dann zum Tragen, wenn einzelsträngige Analyt-DNA oder RNA sehr stark zu stranginterner Sekundärstrukturbildung neigt, so daß räumliche Umorientierungsprozesse die Zugänglichkeit der nachzuweisenden Gruppe beeinflussen. Dies ist für das amplifizierte rpo-β-Gensegment der Fall (siehe FIG 4: 2D-Faltungsvorschlag nach einem Algorithmus von Dr. Zuker, Washington University, Institute for Biomedical Computing). In FIG 5 wurde versucht, die Situation für den Fall bildlich zu illustrieren, in dem (ungleich zu der vorliegenden Erfindung) die Fangsonde direkt mit dem markierten Strang hybridisieren würde, wenn stranginterne Reassoziation (Situation A) bzw. keine stranginterne Reassoziation (Situation B) vorläge.

### SEQUENCE LISTING

<110> Roche Diagnostics GmbH
<120> DNA-Nachweis ueber einen Strangreassoziationskomplex
<130> 4851al
<140>
   <141>
<160> 18
<170> Patent In Ver. 2.0
<210> 1
   <211> 18
   <212> DNA
   <213> Mycobacterium tuberculosis
<400> 1
   tcgccgcgat caaggagt 18
<210> 2
   <211> 19
   <212> DNA
   <213> Mycobacterium tuberculosis
<400> 2
   tgcacgtcgc ggacctcca 19
<210> 3
   <211> 18
   <212> DNA
   <213> Mycobacterium tuberculosis
<220>
   <221> misc_signal
   <222> (1)
   <223> Phosphate ester with digoxigenin
<400> 3
   tcgccgcgat caaggagt 18
<210> 4
   <211> 19
   <212> DNA
   <213> Mycobacterium tuberculosis
<220>
   <221> misc_signal
   <222> (1)
   <223> Phosphate ester with digoxigenin
<400> 4
   tgcacgtcgc ggacctcca 19
<210> 5
   <211> 157
   <212> DNA
   <213> Mycobacterium tuberculosis
<400> 5
   tcgccgcgat caaggagttc ttcggcacca gccagctgag ccaattcatg gaccagaaca 60
   acccgctgtc ggggttgacc cacaagcgcc gactgtcggc gctggggccc ggcggtctgt 120
   cacgtgagcg tgccgggctg gaggtccgcg acgtgca 157
<210> 6
   <211> 38
   <212> DNA
   <213> Mycobacterium tuberculosis
<220>
   <221> misc_signal
   <222> (1)
   <223> Phosphate linked to biotin via Aminolinker
<400> 6
   tttttttttt tttttttttt attggctcag ctggctgg 38
<210> 7
   <211> 38
   <212> DNA
   <213> Mycobacterium tuberculosis
<220>
   <221> misc_signal
   <222> (1)
   <223> Phosphate linked to biotin via Aminolinker
<400> 7
   tttttttttt tttttttttt ttggctcggc tggctggt 38
<210> 8
   <211> 38
   <212> DNA
   <213> Mycobacterium tuberculosis
<220>
   <221> misc_signal
   <222> (1)
   <223> Phosphate linked to biotin via Aminolinker
<400> 8
   tttttttttt tttttttttt gttgttctgg tccatgaa 38
<210> 9
   <211> 38
   <212> DNA
   <213> Mycobacterium tuberculosis
<220>
   <221> misc_signal
   <222> (1)
   <223> Phosphate linked to biotin via Aminolinker
<400> 9
   tttttttttt tttttttttt gttgttctgg agcatgaa 38
<210> 10
   <211> 38
   <212> DNA
   <213> Mycobacterium tuberculosis
<220>
   <221> misc_signal
   <222> (1)
   <223> Phosphate linked to biotin via Aminolinker
<400> 10
   tttttttttt tttttttttt caaccccgac agcgggtt 38
<210> 11
   <211> 38
   <212> DNA
   <213> Mycobacterium tuberculosis
<220>
   <221> misc_signal
   <222> (1)
   <223> Phosphate linked to biotin via Aminolinker
<400> 11
   tttttttttt tttttttttt tcaaccccga cagcgggt 38
<210> 12
   <211> 38
   <212> DNA
   <213> Mycobacterium tuberculosis
<220>
   <221> misc_signal
   <222> (1)
   <223> Phosphate linked to biotin via Aminolinker
<400> 12
   tttttttttt tttttttttt tcggcgcttg tgggtcaa 38
<210> 13
   <211> 38
   <212> DNA
   <213> Mycobacterium tuberculosis
<220>
   <221> misc_signal
   <222> (1)
   <223> Phosphate linked to biotin via Aminolinker
<400> 13
   tttttttttt tttttttttt tcggcgcttg taggtcaa 38
<210> 14
   <211> 38
   <212> DNA
   <213> Mycobacterium tuberculosis
<220>
   <221> misc_signal
   <222> (1)
   <223> Phosphate linked to biotin via Aminolinker
<400> 14
   tttttttttt tttttttttt tcggcgcttg tcggtcaa 38
<210> 15
   <211> 38
   <212> DNA
   <213> Mycobacterium tuberculosis
<220>
   <221> misc_signal
   <222> (1)
   <223> Phosphate linked to biotin via Aminolinker
<400> 15
   tttttttttt tttttttttt tcggcgcttg cgggtcaa 38
<210> 16
   <211> 38
   <212> DNA
   <213> Mycobacterium tuberculosis
<220>
   <221> misc_signal
   <222> (1)
   <223> Phosphate linked to biotin via Aminolinker
<400> 16
   tttttttttt tttttttttt ccccagcgcc gacagtcg 38
<210> 17
   <211> 38
   <212> DNA
   <213> Mycobacterium tuberculosis
<220>
   <221> misc_signal
   <222> (1)
   <223> Phosphate linked to biotin via Aminolinker
<400> 17
   tttttttttt tttttttttt ccccagcgcc aacagtcg 38
<210> 18
   <211> 27
   <212> DNA
   <213> Mycobacterium tuberculosis
<220>
   <221> misc_signal
   <222> (1)
   <223> Phosphate linked to biotin via Aminolinker
<220>
   <221> misc_signal
   <222> (27)
   <223> Y means incorporation of Aminolinker-phosphoramidite subsequently estered with 3-O carboxymethyl digoxigenin
<400> 18
   tttttttttt tttttttttt tttttyt 27

## Patentansprüche

1. Verfahren zum selektiven Nachweis von spezifischen Sequenzen in einer oder mehrerer Nukleinsäure(n) aus einer Probe enthaltend die Schritte
- Amplifikation der Nucleinsäure(n) oder Teilen davon mit Hilfe mindestens eines Sets von zwei Primern, von denen einer mit einem Strang der Nucleinsäure(n) und der andere mit einem hierzu komplementären Strang hybridisieren kann und von denen nur einer ein mit einer nachweisbaren Gruppe markierter Primer ist, unter Bildung von Verlängerungsprodukten dieser Primer für jede dieser Sequenzen,
- Bindung jeweils eines Verlängerungsproduktes an jeweils eine Fangsonde, die dieses Verlängerungsprodukt selektiv binden kann, sofern die Sequenz in der Probe enthalten war, unter Bildung von Hybridisierungskomplexen, wobei die Fangsonden so gewählt werden, daß sie jeweils mit dem Strang des Verlängerungsproduktes hybridisieren können, der eine zu der Sequenz der Fangsonde komplementäre Sequenz enthält und der auch mit dem durch Verlängerung des markierten Primers gebildeten Verlängerungsprodukt hybridisieren kann,
- Abtrennung der an die Fangsonden gebundenen Verlängerungsprodukte von nicht gebundenen Anteilen der Reaktionsmischung, und
- Bestimmung der an die Fangsonden gebundenen nachweisbaren Markierung, **dadurch gekennzeichnet, dass** jeweils der Primer eines Sets von zwei Primern, der die nachweisbare Markierung enthält, das jeweils mit der Fangsonde parallele, nicht direkt hybridisierungsfähige Verlängerungsprodukt erzeugt.

2. Verfahren gemäß Anspruch 1, **dadurch gekennzeichnet, daß** die Fangsonden an räumlich getrennte, unterscheidbare Bereiche einer Festphase gebunden sind.

3. Verfahren gemäß Anspruch 1 oder 2, **dadurch gekennzeichnet, daß** der markierte Primer für die Bildung von Verlängerungsprodukten aus einer Vielzahl von spezifischen Sequenzen verwendet wird.

4. Reagenzkit zum Nachweis von Nucleinsäuren enthaltend in getrennten Behältern
- mindestens ein Set von zwei Primern, von denen nur einer eine nachweisbare Markierung gebunden enthält, zur Amplifikation der Nucleinsäuren oder Teilen davon,
- mindestens 2 immobilisierbare oder immobilisierte Fangsonden unterschiedlicher Sequenz,
**dadurch gekennzeichnet, daß** der markierte Primer so gewählt wird, daß er mit demselben Strang des Verlängerungsproduktes hybridisieren kann wie die Fangsonden.

5. Verwendung eines Reagenzienkits, enthaltend mindestens ein Set von zwei Primern, von denen nur einer ein nachweisbar markierter Primer ist, und mindestens 2 immobilisierte oder immobilisierbare Fangsonden mit unterschiedlicher Sequenz, wobei der nachweisbar markierte Primer so gewählt wird, daß er mit demselben Strang jeder Zielnucleinsäure hybridisieren kann wie die Fangsonden, bei der Mutationsanalyse.

## Claims

1. Method for the selective detection of specific sequences in one or more nucleic acid(s) in a sample comprising the steps
- amplification of the nucleic acid(s) or of parts thereof with the aid of at least one set of two primers one of which can hybridize with one strand of the nucleic acid(s) and the other can hybridize with a complementary strand thereto and only one of which is a primer labelled with a detectable group, to form extension products of these primers for each of these sequences,
- binding in each case one extension product to in each case one capture probe which can selectively bind this extension product provided the sample contained the sequence to form hybridization complexes wherein the capture probes are selected such that they can in each case hybridize with the strand of the extension product which contains a sequence complementary to the sequence of the capture probe and which can also hybridize with the extension product formed by extension of the labelled primer,
- separating the extension products bound to the capture probes from non-bound components of the reaction mixture,
- determining the detectable label bound to the capture probes,
**characterized in that** in each case the primer of a set of two primers which contains the detectable label produces in each case the parallel extension product that cannot directly hybridize with the capture probe.

2. Method according to claim 1, **characterized in that** the capture probes are bound to spatially separate, distinguishable areas of a solid phase.

3. Method according to claim 1 or 2, **characterized in that** the labelled primer is used to form extension products from a plurality of specific sequences.

4. Reagent kit for detecting nucleic acids containing in separate containers
- at least one set of two primers only one of which contains a bound detectable label, for amplifying the nucleic acids or parts thereof,
- at least two immobilizable or immobilized capture probes of different sequence,
**characterized in that** the labelled primer is selected such that it can hybridize with the same strand of the extension product as the capture probes.

5. Use of a reagent kit containing at least one set of two primers only one of which is a detectably labelled primer, and at least two immobilized or immobilizable capture probes having different sequences, wherein the detectably labelled primer is selected such that it can hybridize with the same strand of each target nucleic acid as the capture probes in a mutation analysis.

## Revendications

1. Procédé pour le décèlement sélectif de séquences spécifiques dans un ou plusieurs acides nucléiques à partir d'un échantillon, contenant les étapes
- de l'amplification d e l'acide/des a cides nucléiques ou de parties de ce(s) dernier(s) à l'aide d'au moins un jeu de deux amorces dont l'une peut s'hybrider avec un brin de l'acide/des acides nucléiques et l'autre peut s'hybrider avec un brin complémentaire au premier cité et dont seulement une est une amorce marquée avec un groupe décelable, avec formation de produits d'extension de ces amorces pour chacune de ces séquences,
- de la liaison respectivement d'un produit d'extension à respectivement une sonde de fixation qui peut se lier de manière sélective à ce produit d'extension, pour autant que la séquence ait été contenue dans la sonde, avec formation de complexes d'hybridation, les sondes de fixation étant sélectionnées de telle sorte qu'elles peuvent s'hybrider respectivement avec le brin du produit d'extension qui contient une séquence complémentaire à la séquence de la sonde de fixation et qui peut s'hybrider également avec le produit d'extension formé par l'extension de l'amorce marquée,
- de la séparation des produits d'extension liés aux sondes de fixation par rapport aux fractions non liées du mélange réactionnel, et
- de la détermination du marqueur décelable lié aux sondes de fixation, **caractérisé en ce que** respectivement l'amorce d'un jeu de deux amorces, qui contient le marqueur décelable, génère le produit d'extension, respectivement parallèle à la sonde de fixation, inapte à une hybridation directe.

2. Procédé selon 1 a revendication 1 , **caractérisé** e n ce que les sondes d e fixation sont liées à des zones discernables d'une phase solide, séparées dans l'espace.

3. Procédé selon la revendication 1 ou 2, **caractérisé en ce que** l'amorce marquée est utilisée pour la formation de produits d'extension à partir d'une multitude de séquences spécifiques.

4. Nécessaire de réactif pour le décèlement d'acides nucléiques contenant, dans des récipients séparés,
- au moins un jeu de deux amorces dont seulement une contient, à l'état lié, un marqueur décelable, pour l'amplification des acides nucléiques ou de parties de ces derniers,
- au moins deux sondes de fixation de séquences différentes, immobilisables ou immobilisées,
**caractérisé en ce que** l'amorce marquée est sélectionnée de telle sorte qu'elle peut s'hybrider avec le même brin du produit d'extension que celui auquel s'hybrident les sondes de fixation.

5. Utilisation d'un nécessaire de réactifs, contenant au moins un jeu de deux amorces, dont seulement une est une amorce marquée décelable, et au moins deux sondes de fixation immobilisées ou immobilisables, contenant des séquences différentes, la sonde marquée décelable étant sélectionnée de telle sorte qu'elle peut s'hybrider avec le même brin de chaque acide nucléique cible que celui auquel s'hybrident les sondes de fixation lors de l'analyse des mutations.
